(19) Europäisches Patentamt
European Patent Office
Office européen des brevets

(11) EP 4 459 336 A1

(12) **EUROPEAN PATENT APPLICATION**
published in accordance with Art. 153(4) EPC

(43) Date of publication:
**06.11.2024 Bulletin 2024/45**

(21) Application number: **22916102.1**

(22) Date of filing: **27.12.2022**

(51) International Patent Classification (IPC):
***G02B 1/04*** *(2006.01)* ***C08G 75/08*** *(2006.01)*
***G02C 7/00*** *(2006.01)*

(52) Cooperative Patent Classification (CPC):
**C08G 75/08; G02B 1/04; G02C 7/00**

(86) International application number:
**PCT/JP2022/048205**

(87) International publication number:
**WO 2023/127880 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021212629**

(71) Applicant: **Hoya Lens Thailand Ltd.**
**Pathumthani 12130 (TH)**

(72) Inventor: **KOUSAKA, Masahisa**
**Tokyo 160-8347 (JP)**

(74) Representative: **Kraus & Lederer PartGmbB**
**Thomas-Wimmer-Ring 15**
**80539 München (DE)**

(54) **POLYMERIZABLE COMPOSITION FOR OPTICAL COMPONENTS, CURED OBJECT, AND SPECTACLE LENS**

(57) One embodiment according to the present disclosure relates to a polymerizable composition for optical components, including an epithio compound (1) having an ether bond and an epithio compound (2) having a thioether bond or a dithioether bond.

EP 4 459 336 A1

## Description

[Technical Field]

The present disclosure relates to a polymerizable composition for optical components, a cured object, and a spectacle lens.

[Background Art]

Spectacle lenses made of plastics have conventionally been known. As materials for spectacle lenses, polythiourethane resins are used (for example, PTL 1). Polythiourethane resins have been widely spread because they are materials having moderate refractive indexes and can provide spectacle lenses with moderate thicknesses, and processability and impact resistance are easy to ensure.

[Citation List]

[Patent Literature]

[PTL 1] Japanese Patent Application Publication No. 2004-002820

[Summary of Invention]

[Technical Problem]

Epithio resins have generally been known as materials with high refractive indexes and Abbe's numbers. Epithio resin has high refractive indexes and thus can provide thin spectacle lenses. Meanwhile, a relatively thick lens may be required in some cases in consideration of processability and impact resistance. For example, a problem of resins for optical components, such as polythiourethane resins, is that when the refractive index is lower, the Abbe's number is lower than that of epithio resins. The Abbe's number is an index that expresses the degree of dependence of the refractive index on the light wavelength, and a higher Abbe's number can reduce the chromatic aberration of a spectacle lens.

One embodiment of the present disclosure relates to a polymerizable composition for optical components that can provide a cured object with a moderate refractive index and a high Abbe's number, a cured object thereof, and a spectacle lens including the cured object.

[Solution to Problem]

The present inventor has found that the use of a compound represented by formula (1) and a compound represented by formula (2) as a polymerizable composition for optical components can provide a cured object with a moderate refractive index and a high Abbe's number.

One embodiment according to the present disclosure relates to a polymerizable composition for optical components, including

an epithio compound (1) having an ether bond and
an epithio compound (2) having a thioether bond or a dithioether bond.

The present inventor has found that the use of an epithio compound (1) with a refractive index of less than 1.55 and an epithio compound (2) with a refractive index of 1.60 or more as a polymerizable composition for optical components can provide a cured object with a moderate refractive index and a high Abbe's number.

One embodiment according to the present disclosure relates to a polymerizable composition for optical components, including

an epithio compound (1') with a refractive index of less than 1.55 and
an epithio compound (2') with a refractive index of 1.60 or more.

[Advantageous Effects of Invention]

According to one embodiment of the present disclosure, a polymerizable composition for optical components that can provide a cured object with a moderate refractive index and a high Abbe's number, a cured object thereof, and

a spectacle lens including the cured object can be provided.

[Description of Embodiments]

Hereinafter, embodiments of the present disclosure (hereinafter referred to as "the present embodiment") will be described in detail, but the present invention is not limited thereto, and can be variously modified within the scope not departing from the gist of the invention. In the present description, for example, the notation of a numerical range of "1 to 100" shall encompass both the lower limit "1" and the upper limit "100". In addition, the same applies to the notation of other numerical ranges.

[Polymerizable Composition for Optical Components]

One embodiment according to the present disclosure relates to a polymerizable composition for optical components, including

an epithio compound (1) having an ether bond and
an epithio compound (2) having a thioether bond or a dithioether bond.

The polymerizable composition for optical components according to one embodiment of the present disclosure can provide a cured object with a moderate refractive index and a high Abbe's number.

A polythiourethane resin is used as a material for spectacle lenses because a polythiourethane resin has a refractive index of 1.64 or more and 1.68 or less and is highly heat resistant. Polythiourethane resins have been widely spread because they are materials with refractive indexes of 1.64 or more and 1.68 or less and can provide spectacle lenses with a moderate thickness, and processability and impact resistance are easy to ensure. For example, polythiourethane resins have an issue of low Abbe's numbers, although moderate refractive indexes as 1.64 or more and 1.68 or less can be obtained. Abbe's number is an index that expresses the degree of dependence of the refractive index on the light wavelength, and a higher Abbe's number can reduce the chromatic aberration of a spectacle lens.

One embodiment of the present disclosure relates to a polymerizable composition for optical components that can provide a cured object with a refractive index of 1.64 or more and 1.68 or less and a high Abbe's number, a cured object thereof, and a spectacle lens including the cured object. A polymerizable composition for optical components of one embodiment of the present invention includes

a compound represented by formula (1):

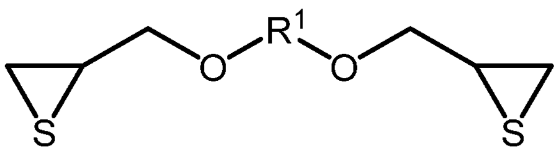

(1)

(wherein $R^1$ is a divalent C1-20 aliphatic hydrocarbon group) and
a compound represented by formula (2):

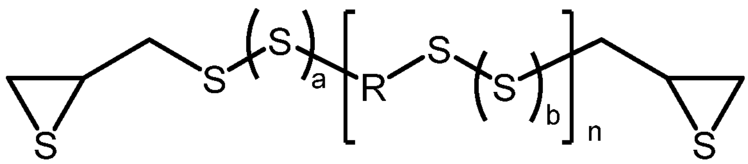

(2)

(wherein a is an integer of 0 or 1, R is a divalent divalent C1-10 hydrocarbon group, b is an integer of 0 or 1, and n is an integer of 0 to 2).
The polymerizable composition for optical components according to one embodiment of the present disclosure can provide a cured object with a refractive index of 1.64 or more and 1.68 or less and a high Abbe's number.

<Epithio Compound (1)>

The epithio compound (1) has ether bonds. The number of ether bonds in the epithio compound (1) is preferably

1 to 5, more preferably 1 to 3, and still more preferably 2.

The epithio compound (1) is preferably

a compound represented by formula (1):

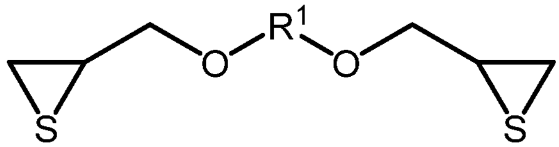

( 1 )

(wherein $R^1$ is a divalent C1-20 aliphatic hydrocarbon group).

The aliphatic hydrocarbon group $R^1$ may be any of linear, branched, and cyclic, and a linear one is preferred. The number of carbon atoms in $R^1$ is preferably 1 to 10, more preferably 1 to 6, and still more preferably 1 to 3. Examples of divalent aliphatic hydrocarbons for $R^1$ include a methylene group, an ethylenediyl group, and a propanediyl group.

The epithio compound (1) is preferably a compound represented by formula (1-1):

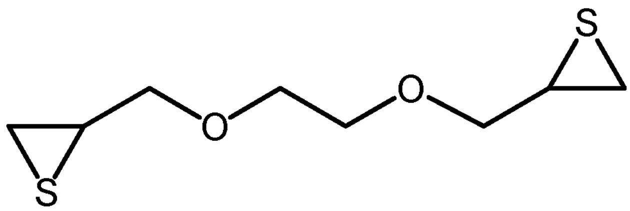

( 1-1 )

(ethylene glycol bis(β-epithiopropyl) ether; hereinafter referred to as compound (1-1)).

The refractive index of the epithio compound (1) is preferably less than 1.55, more preferably 1.54 or less, and further preferably 1.53 or less. The refractive index of an epithio compound (1) herein means that of an epithio compound (1) before polymerization. The refractive index is measure by d line (587.6 nm). The refractive index is measured according to the measurement method in the Examples. Hereinafter, the refractive indexes of various epithio compounds shall be measured by the same method.

The content of the epithio compound (1) is preferably 5% to 95% by mass, more preferably 10% to 80% by mass, further preferably 25% to 75% by mass, and still more preferably 35% to 60% by mass in relation to the total amount of the polymerizable composition for optical components.

<Epithio Compound (2)>

The epithio compound (2) has thioether bonds or dithioether bonds. The number of thioether bonds or dithioether bonds in the epithio compound (2) is preferably 1 to 5, more preferably 1 to 3, and still more preferably 1. It is noted that a thioether bond means a chemical structure represented by *-S-* (* denotes a binding site). A dithioether bond means *-SS-* (* denotes a binding site).

The epithio compound (2) is preferably

a compound represented by formula (2):

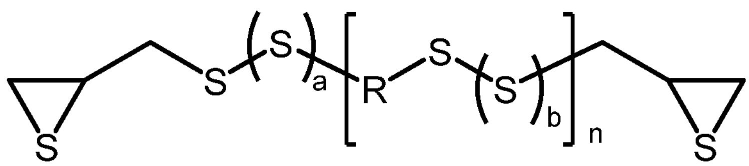

( 2 )

(wherein a is an integer of 0 or 1, R is a divalent C1-10 hydrocarbon group, b is an integer of 0 or 1, and n is an integer of 0 to 2).

The number of carbon atoms in R is preferably 1 to 4 and more preferably 2 or 3.

Examples of divalent hydrocarbons for R include an ethylene group, a propylene group, and a butylene group.
Specific examples of the epithio compounds (2) include bis(β-epithiopropyl) sulfide, bis(β-epithiopropyl) dithioether, bis(β-epithiopropyl) trisulfide, bis(β-epithiopropylthio)methane, 1,2-bis(β-epithiopropylthio)ethane, 1,3-bis(β-epithiopropylthio)propane, 1,3-bis(β-epithiopropyloxy)propane, 1,4-bis(β-epithiopropylthio)butane, and bis(β-epithiopropylthioethyl) sulfide. Among these, bis(β-epithiopropyl) sulfide and bis(β-epithiopropyl) dithioether are preferable, and bis(β-epithiopropyl) sulfide is more preferable. That is, the epithio compound (2) is preferably a compound represented by the following formula (2-1) :

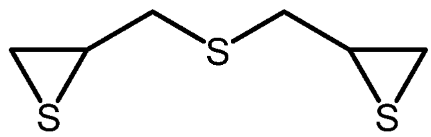

( 2-1 )

The refractive index of the epithio compound (2) is preferably 1.60 or more and more preferably 1.61 or more.
The difference in refractive index between the epithio compound (1) and the epithio compound (2) is preferably less than 0.10, more preferably 0.09 or less, and further preferably 0.08 or less. When the difference in refractive index is less than 0.10, the occurrence of cloudiness during polymerization can be suppressed. The difference in refractive index between the epithio compound (1) and the epithio compound (2) is preferably 0.01 or more, more preferably 0.03 or more, and further preferably 0.05 or more.
The content of the epithio compound (2) is preferably 5% to 95% by mass, more preferably 10% to 80% by mass, further preferably 25% to 75% by mass, and still more preferably 40% to 65% by mass in relation to the total amount of the polymerizable composition for optical components.
The total content of the epithio compound (1) and the epithio compound (2) is preferably 80% by mass or more, more preferably 90% by mass or more, or 95% by mass or more, in relation to the total amount of the polymerizable composition for optical components.
One embodiment according to the present disclosure relates to a polymerizable composition for optical components, including

an epithio compound (1') with a refractive index of less than 1.55 and
an epithio compound (2') with a refractive index of 1.60 or more.

The polymerizable composition for optical components according to one embodiment of the present disclosure can provide a cured object with a refractive index of 1.64 or more and 1.68 or less and a high Abbe's number.

<Epithio Compound (1')>

The epithio compound (1') has a refractive index of less than 1.55. The refractive index of the epithio compound (1') is preferably 1.54 or less and more preferably 1.53 or less. Examples of the epithio compound (1') include those listed for the epithio compound (1) described above.
The content of the epithio compound (1') is preferably 5% to 95% by mass, more preferably 10% to 80% by mass, further preferably 25% to 75% by mass, and still more preferably 35% to 60% by mass in relation to the total amount of the polymerizable composition for optical components.

<Epithio Compound (2')>

The epithio compound (2') has a refractive index of 1.60 or more. The refractive index of the epithio compound (2') is preferably 1.61 or more.
Examples of the epithio compound (2') include those listed for the epithio compound (2) described above.
The content of the epithio compound (2') is preferably 5% to 95% by mass, more preferably 10% to 80% by mass, further preferably 25% to 75% by mass, and still more preferably 40% to 65% by mass in relation to the total amount of the polymerizable composition for optical components.
The difference in refractive index between the epithio compound (1') and the epithio compound (2') is preferably less than 0.10, more preferably 0.09 or less, and further preferably 0.08 or less. When the difference in refractive index is less than 0.10, the occurrence of cloudiness during polymerization can be suppressed. The difference in refractive index between the epithio compound (1') and the epithio compound (2') is preferably 0.01 or more, more preferably 0.03 or more, and further preferably 0.05 or more.
The total content of the epithio compound (1') and the epithio compound (2') is preferably 80 by mass or more,

90% by mass or more, or 95% by mass or more in relation to the total amount of the polymerizable composition for optical components.

The polymerizable composition for optical components may contain other monomers. Examples of other monomers include polythiol compounds.

Examples of polythiol compounds include an ester compound of a polyol compound with a mercapto group-containing carboxylic acid compound, a linear or branched aliphatic polythiol compound, a polythiol compound having an alicyclic structure, and a polythiol compound having an aromatic ring structure.

In the ester compound of a polyol compound with a mercapto group-containing carboxylic acid compound, examples of the polyol compound include a compound having two or more hydroxyl groups in its molecule.

Examples of polyol compounds include ethylene glycol, diethylene glycol, propanediol, propanetriol, butanediol, trimethylolpropane, bis(2-hydroxyethyl) dithioether, pentaerythritol, and dipentaerythritol.

Examples of mercapto group-containing carboxylic acid compounds include thioglycolic acid, mercaptopropionic acid, a thiolactic acid compound, and thiosalicylic acid.

Examples of the ester compound of a polyol compound with a mercapto group-containing carboxylic acid compound include ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tris(2-mercaptoacetate), trimethylolpropane tris(3-mercaptopropionate), pentaerythritol tetrakis(2-mercaptoacetate), pentaerythritol tetrakis(3-mercaptopropionate), dipentaerythritol hexakis(2-mercaptoacetate), and dipentaerythritol hexakis(3-mercaptopropionate).

Examples of linear or branched aliphatic polythiol compounds include 1,2-ethanedithiol, 1,1-propanedithiol, 1,2-propanedithiol, 1,3-propanedithiol, 2,2-propanedithiol, 1,6-hexanedithiol, 1,2,3-propanetrithiol, 2,2-dimethylpropane-1,3-dithiol, 3,4-dimethyloxybutane-1,2-dithiol, 2,3-dimercapto-1-propanol, 1,2-dimercaptopropyl methyl ether, 2,3-dimercaptopropyl methyl ether, 2-(2-mercaptoethylthio)propane-1,3-dithiol, 2,2-bis(mercaptomethyl)-1,3-propanedithiol, bis(mercaptomethylthio)methane, tris(mercaptomethylthio)methane, bis(2-mercaptoethylthio)methane, 1,2-bis(mercaptomethylthio)ethane, 1,2-bis(2-mercaptoethylthio)ethane, 1,3-bis(mercaptomethylthio)propane, 1,3-bis(2-mercaptoethylthio)propane, 1,1,2,2-tetrakis(mercaptoethylthio)ethane, 1,1,3,3-tetrakis(mercaptoethylthio)propane, 3-mercaptomethyl-1,5-dimercapto-2,4-dithiapentane, tetrakis(mercaptoethylthio)propane, bis(2-mercaptoethyl) ether, bis(2-mercaptoethyl) sulfide, bis(2-mercaptoethyl) dithioether, 1,2-bis(2-mercaptoethylthio)-3-mercaptopropane, 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

Examples of polythiol compounds having an aliphatic ring structure include 1,1-cyclohexanedithiol, 1,2-cyclohexanedithiol, methylcyclohexanedithiol, bis(mercaptomethyl)cyclohexane, 2-(2,2-bis(mercaptomethylthio)ethyl)-1,3-dithiethane, 2,5-bis(mercaptomethyl)-1,4-dithiane, and 4,8-bis(mercaptomethyl)-1,3-dithiane.

Examples of polythiol compounds having an aromatic ring structure include 1,3-dimercaptobenzene, 1,4-dimercaptobenzene, 1,3-bis(mercaptomethyl)benzene, 1,4-bis(mercaptomethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptoethyl)benzene, 4,4'-dimercaptobiphenyl, 4,4'-dimercaptobibenzyl, 2,5-toluenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methyloxyphenyl)propane-2,2-dithiol, 1,3-diphenylpropane-2,2-dithiol, phenylmethane-1,1-dithiol, and 2,4-di(p-mercaptophenyl)pentane.

These may be used alone, or two or more thereof may be used.

The content of the polythiol compound is preferably 2% by mass or more and more preferably 4% by mass or more, and preferably 50% by mass or less, more preferably 40% by mass or less, still more preferably 30% by mass or less, even more preferably 20% by mass or less, and further preferably 10% by mass or less in the polymerizable composition for optical components.

The polymerizable composition for optical components preferably contains a polymerization catalyst. Examples of polymerization catalysts include a nitrogen-containing compound.

Examples of nitrogen-containing compounds include tertiary amines, quaternary ammonium salts, imidazole compounds, and pyrazole compounds. The tertiary amines are preferably hindered amines.

Examples of tertiary amines include triethylamine, tri-n-propylamine, triisopropylamine, tri-n-butylamine, triisobutylamine, N,N-dimethylbenzylamine, N-methylmorpholine, N,N-dimethylcyclohexylamine, pentamethyldiethylenetriamine, bis(2-dimethylaminoethyl) ether, N-methylmorpholine, N,N'-dimethylpiperazine, N,N,N',N'-tetramethylethylenediamine, and 1,4-diazabicyclo[2.2.2]octane (DABCO).

Examples of hindered amines include 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, a mixture of methyl 1,2,2,6,6-pentamethyl-4-piperidyl sebacate and bis(1,2,2,6,6-pentamethyl-4-piperidyl) sebacate, bis(1,2,2,6,6-pentamethyl-4-piperidyl) sebacate, bis(2,2,6,6-tetramethyl-1-(octyloxy)-4-piperidyl) sebacate, bis(1,2,2,6,6-pentamethyl-4-piperidyl) [[3,5-bis(1,1-dimethylethyl)-4-hydroxyphenyl]methyl]butyl malonate, and tetrakis(1,2,2,6,6-pentamethyl-4-piperidyl)butane-1,2,3,4-tetracarboxylate.

Examples of quaternary ammonium salts include tetraethylammonium hydroxide.

Examples of imidazole compounds include imidazole, 1-methyl-2-mercapto-1H-imidazole, 1,2-dimethylimidazole, benzylmethylimidazole, and 2-ethyl-4-imidazole.

Examples of pyrazole compounds include pyrazole and 3,5-dimethylpyrazole.

Among these, tertiary amines such as hindered amines, imidazole compounds, and pyrazole compounds are preferable, imidazole compounds are more preferable, and 1-methyl-2-mercapto-1H-imidazole is still more preferable.

The amount of the polymerization catalyst added in the polymerizable composition is preferably 0.001 parts by mass or more, more preferably 0.005 parts by mass or more, and still more preferably 0.007 parts by mass or more, and preferably 2 parts by mass or less, more preferably 1 part by mass or less, and still more preferably 0.5 parts by mass or less in relation to the total amount of 100 parts by mass of the polymerizable component.

[Cured Object]

A polymerizable composition for optical components according to the present embodiment is used as a cured object. The refractive index nd of the cured polymerizable composition for optical components is preferably 1.64 to 1.68. The refractive index nd of a cured object is a value measured by d line (587.6 nm). The refractive index nd is measured according to the measurement method in the Examples.

The Abbe's number vd of the cured polymerizable composition for optical components may be, for example, 37 or more, 38 or more, 39 or more, and 43 or more. The Abbe's number vd is measured according to the measurement method in the Examples.

It is preferred that the thermal expansion characteristic of the cured object is linear. When the thermal expansion characteristic is linear, cracks are less likely to occur when a coating layer or the like is formed on the surface of a cured object.

Examples of applications of the cured object of the present embodiment include spectacle lenses (materials for lens substrates), prisms, optical fibers, information recording bases, and filters. Among these applications, spectacle lenses are preferable, and lens substrates are more preferable. Heat curing or photocuring may be adopted as a method for producing the cured object.

[Lens Substrate and Spectacle Lens]

A lens substrate used for spectacle lenses may be either a finished lens or a semi-finished lens. The surface shape of the lens substrate is not particularly limited and may be any of a flat surface, a convex surface, a concave surface, or the like.

The lens substrate may be any of a single vision lens, a multifocal lens, a progressive power lens, or the like. For example, as an example, regarding a progressive power lens, a near vision region (near portion) and a progressive vision region (intermediate region) are normally included in the lower region described above, and a distance vision region (distance portion) is normally included in an upper region. As the lens substrate, a colorless substrate is usually used, but a colored one can be used to the extent that transparency is not impaired.

The thickness and diameter of the lens substrate are not particularly limited, and the thickness is normally 1 to 30 mm, and the diameter is normally 50 to 100 mm. The refractive index of the lens substrate is preferably 1.64 to 1.68.

The lens substrate includes the cured object described above.

[Method for Producing Lens Substrate]

Although not particularly limited, the lens substrate is obtained by, for example, a method including

a step of curing the polymerizable composition for optical components described above and
a step of annealing a cured resin.

The polymerization is preferably performed by a cast polymerization method. A lens substrate can be obtained by injecting the polymerizable composition for optical components into a molding die, which is a combination of a glass or metal mold and a tape or a gasket, and performing polymerization.

Polymerization conditions can be set, as appropriate, according to the polymerizable composition for optical components. The polymerization starting temperature is preferably 0°C or higher and more preferably 10°C or higher, and preferably 50°C or lower and more preferably 40°C or lower. It is preferable to raise the temperature from the polymerization starting temperature, and then perform heating, curing, and shaping. For example, the raised maximum temperature is generally 110°C or higher and 130°C or lower.

After polymerization is completed, the lens substrate may be de-molded and subjected to an annealing treatment. The temperature of the annealing treatment is preferably 100°C to 150°C.

As described above, the present disclosure can provide a polymerizable composition for optical components that can provide a cured object with a moderate refractive index and a high Abbe's number, a cured object thereof, and a spectacle lens including the cured object. The cured object has a refractive index of within the range of 1.64 to 1.68 and has a high Abbe's number and is therefore suitably used as a substrate used for spectacle lenses.

The present disclosure discloses the following embodiments.

<1> A polymerizable composition for optical components, including

an epithio compound (1) having an ether bond and
an epithio compound (2) having a thioether bond or a dithioether bond.

<2> The polymerizable composition for optical components according to <1>, wherein a content of the epithio compound (1) is 5% to 95% by mass in relation to the total amount of the polymerizable composition for optical components.

<3> The polymerizable composition for optical components according to <1> or <2>, wherein a content of the epithio compound (2) is 5% to 95% by mass in relation to the total amount of the polymerizable composition for optical components.

<4> The polymerizable composition for optical components according to any one of <1> to <3>, wherein the epithio compound (1) is a compound represented by formula (1):

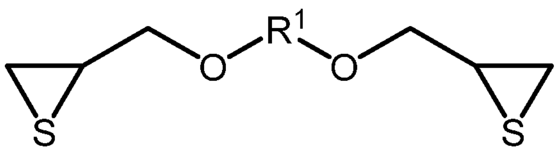

( 1 )

(wherein $R^1$ is a divalent C1-20 aliphatic hydrocarbon group).

<5> The polymerizable composition for optical components according to <4>, wherein the epithio compound (1) is a compound represented by
formula (1-1):

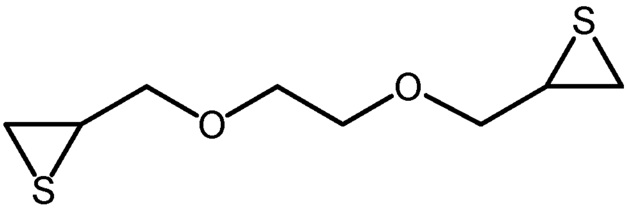

( 1-1 )

.

<6> The polymerizable composition for optical components according to any one of <1> to <5>, wherein the epithio compound (2) is a compound represented by
formula (2):

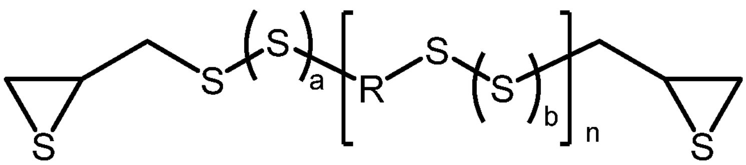

( 2 )

(wherein a is an integer of 0 or 1, R is a divalent C1-10 hydrocarbon group, b is an integer of 0 or 1, and n is an integer of 0 to 2).

<7> The polymerizable composition for optical components according to <6>, wherein the epithio compound (2) is a compound represented by
formula (2-1):

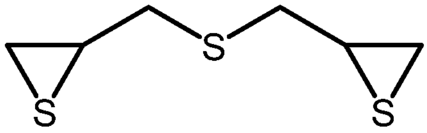
( 2-1 )

.

<8> The polymerizable composition for optical components according to any one of <1> to <7>, wherein

the epithio compound (1) has a refractive index of less than 1.55 and
the epithio compound (2) has a refractive index of 1.60 or more.

<9> The polymerizable composition for optical components according to <8>, wherein a difference in refractive index between the epithio compound (1) and the epithio compound (2) is less than 0.10.
<10> A polymerizable composition for optical components including:

an epithio compound (1') with a refractive index of less than 1.55 and
an epithio compound (2') with a refractive index of 1.60 or more.

<11> The polymerizable composition for optical components according to <10>, wherein the polymerizable composition for optical components has a difference in refractive index between the epithio compound (1') and the epithio compound (2') of less than 0.10.
<12> The polymerizable composition for optical components according to any one of <1> to <11>, wherein the polymerizable composition for optical components after being cured has a refractive index of 1.64 to 1.68.
<13> The polymerizable composition for optical components according to any one of <1> to <12>, wherein the polymerizable composition for optical components after being cured has an Abbe's number of 37 or larger.
<14> A cured object of the polymerizable composition for optical components according to any one of <1> to <13>.
<15> A spectacle lens including a lens substrate that includes the cured object according to <14>.

[Examples]

Hereinafter, the present embodiment will be described in more detail with reference to Examples and Comparative Examples. The present invention is not limited to the following Examples.

[Measurement Method]

<Refractive Index and Abbe's Number>

The refractive index of a cured object or a compound was measured using a precision refractometer "KPR-2000" (manufactured by Kalnew Optical Industries) at 25°C with F line (486.1 nm), C line (656.3 nm), and d line (587.6 nm). Then, the Abbe's number was calculated from the following formula.

$$\text{Abbe's number } \nu d = (nd-1)/(nF-nC)$$

The nd denotes a refractive index measured with the d line, nF denotes a refractive index measured with the F line, and nC represents a refractive index measured with the C line.

<Thermal Expansion Characteristic>

Thermal expansion characteristics were measured by a penetration method (sample thickness: 3 mm, pin diameter: 0.5 mm, loaded weight: 10 g, and temperature rising speed: 10°C/min) using a thermomechanical analyzer, "Thermo Plus EVO2 TMA8310" (manufactured by Rigaku Corporation) while rising the temperature from room temperature (25°C) to 150°C. When no peaks and inflection points are observed in thermal expansion characteristics, the characteristics are regarded as "linear", and when any peaks or inflection points are observed, the characteristics are regarded as "non-linear".

<Absorption Peak within UV Region>

Absorption peaks within the UV region were observed by measurement using a spectrophotometer "U-4100" (product name, manufactured by Hitachi, Ltd.). The point to be measured of the absorption peaks within the UV region is set to the optical center of a spectacle lens.

<Production Example 1>

First, 87.00 g of ethylene glycol diglycidyl ether and 76.12 g of thiourea were dissolved in 250 mL of toluene and 250 mL of methanol, then 1.50 g of acetic acid was added to prepare a reaction mixture solution, and a reaction was caused at a room temperature (25°C) for 18 hours.
To the reaction mixture solution, 500 mL of toluene was added, then 500 mL of water containing 1.00 g of dissolved NaCl was added, the aqueous layer and an organic layer were separated, the organic layer was then taken out, and the organic layer was washed with 300 mL of 1% by mass aqueous sulfuric acid solution. Washing an organic layer with 330 mL of water was repeated seven times, the organic layer was taken out, and toluene and the water content were removed by distillation to prepare a colorless and transparent ethylene glycol bis(β-epithiopropyl) ether (refractive index (nd; 25°C) = 1.53).

<Example 1>

In a 300-ml eggplant flask, 50.00 g of bis(β-epithiopropyl) sulfide (refractive index (nd; 25°C) = 1.61), 50.00 g of ethylene glycol bis(β-epithiopropyl) ether, 5.00 g of bismercaptoethyl dithiane, 0.15 g of tetrabutylphosphonium bromide as a catalyst, and 0.10 g of 2-(2-hydroxy-4-octyloxyphenyl)-2H-benzotriazole as a UV ray absorber were fed and then mixed and dissolved to homogenize. Degassing was performed while stirring to remove gases contained during mixing. The resulting mixture was injected into a lens mold (set at 0.00D center wall thickness of 1.8 mm and -4.00D center wall thickness of 1.0 mm) consisting of a glass mold and a resin gasket while filtered through a polytetrafluoroethylene (PTFE) filter with a pore diameter of 0.45 microns, and polymerization was performed in an electric furnace at a temperature from 20°C to 110°C over 24 hours. After completion of the polymerization, the gasket and the mold were removed, followed by heat treatment for 2 hours at 105°C to prepare a lens substrate. The optical properties of the resulting lens substrate were measured. Table 1 shows the results.

<Examples 2 and 3>

A resin composition was obtained in the same additive formulation and operation as in Example 1, except that the formulation amounts of bis(β-epithiopropyl) sulfide and ethylene glycol bis(β-epithiopropyl) ether followed the composition in Table 1.

<Comparative Example 1>

A resin composition was obtained in the same additive formulation and operation as in Example 1, except that 100.00 g of bis(β-epithiopropyl) sulfide was added, but ethylene glycol bis(β-epithiopropyl) ether was not formulated.

<Comparative Example 2>

First, 50.60 g of xylylene diisocyanate as an isocyanate compound, 0.10 g of 2-(2-hydroxy-4-octyloxyphenyl)-2H-benzotriazole as a UV ray absorber, 0.14 g of a 4:6 mixture of monobutoxyethyl acid phosphate and dibutoxyethyl acid phosphate as a mold release agent, and 0.01 g of dimethyltin dichloride as a catalyst were fed and then mixed to dissolve. Then, a curable composition was prepared by adding 49.40 g of a structural isomer mixture of 4,7-bis(mercaptomethyl)-3,6,9-trithiaundecane-1,11-diol, and the resulting mixture was stirred and mixed for 30 minutes under a reduced pressure of 666 Pa (5 mmHg). Next, this curable composition was injected into the same lens mold consisting of a glass mold and a resin gasket used in the Examples while filtrating this curable composition through a PTFE filter with a pore diameter of 5 microns, and polymerization was performed in an electric furnace at a temperature of from 20°C to 120°C over 24 hours. After completion of the polymerization, the gasket and the mold were removed, followed by heat treatment for 2 hours at 120°C to prepare a lens substrate. The optical properties of the resulting lens substrate were measured. Table 1 shows the results.

Table 1

| | | | Example 1 | Example 2 | Example3 | Comp. Example 1 | Comp. Example 2 |
|---|---|---|---|---|---|---|---|
| Polymerizable composition (parts by mass) | Compound (1) | | 50 | 25 | 95 | 0 | Thiourethane |
| | Compound (2) | | 50 | 75 | 5 | 100 | |
| Characteristics of cured object | Refractive index | nF | 1.654 | 1.684 | 1.625 | 1.721 | 1.679 |
| | | nd | 1.643 | 1.671 | 1.615 | 1.707 | 1.664 |
| | | nC | 1.638 | 1.666 | 1.611 | 1.701 | 1.658 |
| | Abbe's number | *ν* d | 39 | 37 | 47 | 36 | 32 |
| | Thermal expansion characteristic | | Linear | Linear | Linear | Linear | Non-Linear |

From the above results, the polymerizable compositions of the Examples show high Abbe's numbers while having a moderate refractive index. Furthermore, because a cured object of the polymerizable composition according to the Examples has a linear thermal expansion characteristic, lamination of other layers on the surface of a lens substrate, or the like, is easier.

## Claims

**1.** A polymerizable composition for optical components, comprising:

an epithio compound (1) having an ether bond and
an epithio compound (2) having a thioether bond or a dithioether bond.

**2.** The polymerizable composition for optical components according to claim 1, wherein a content of the epithio compound (1) is 5% to 95% by mass in relation to a total amount of the polymerizable composition for optical components.

**3.** The polymerizable composition for optical components according to claim 1, wherein a content of the epithio compound (2) is 5% to 95% by mass in relation to a total amount of the polymerizable composition for optical components.

**4.** The polymerizable composition for optical components according to claim 1, wherein the epithio compound (1) is a compound represented by
formula (1):

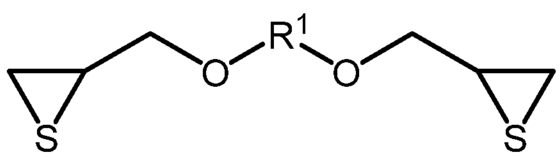

(1)

(wherein $R^1$ is a divalent C1-20 aliphatic hydrocarbon group).

**5.** The polymerizable composition for optical components according to claim 4, wherein the epithio compound (1) is a compound represented by
formula (1-1):

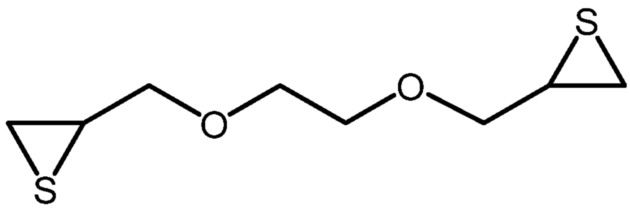

( 1-1 )

.

6. The polymerizable composition for optical components according to claim 1, wherein the epithio compound (2) is a compound represented by formula (2):

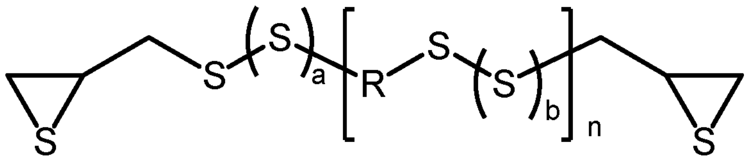

( 2 )

(wherein a is an integer of 0 or 1, R is a divalent C1-10 hydrocarbon group, b is an integer of 0 or 1, and n is an integer of 0 to 2).

7. The polymerizable composition for optical components according to claim 6, wherein the epithio compound (2) is a compound represented by formula (2-1):

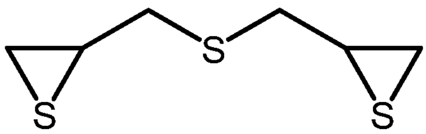

( 2-1 )

.

8. The polymerizable composition for optical components according to claim 1, wherein

the epithio compound (1) is a compound represented by formula (1):

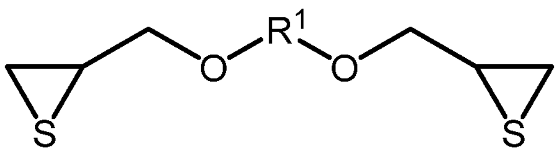

( 1 )

(wherein $R^1$ is a divalent C1-20 aliphatic hydrocarbon group) and the epithio compound (2) is a compound represented by formula (2):

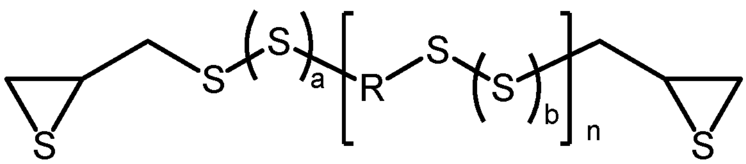

( 2 )

(wherein a is an integer of 0 or 1, R is a divalent C1-10 hydrocarbon group, b is an integer of 0 or 1, and n is an integer of 0 to 2).

9. The polymerizable composition for optical components according to claim 1, wherein the epithio compound (1) is a compound represented by formula (1-1):

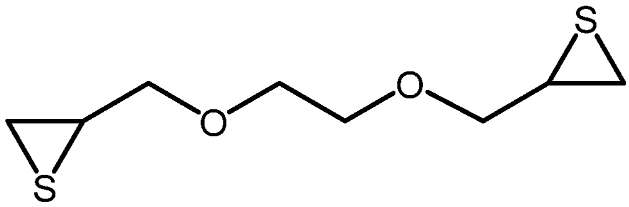

( 1-1 )

and

the epithio compound (2) is a compound represented by formula (2-1):

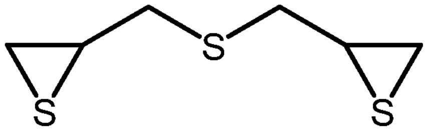

( 2-1 )

.

**10.** The polymerizable composition for optical components according to claim 1, wherein

the epithio compound (1) has a refractive index of less than 1.55 and
the epithio compound (2) has a refractive index of 1.60 or more.

**11.** The polymerizable composition for optical components according to claim 10, wherein a difference in refractive index between the epithio compound (1) and the epithio compound (2) is less than 0.10.

**12.** A polymerizable composition for optical components comprising:

an epithio compound (1') with a refractive index of less than 1.55 and
an epithio compound (2') with a refractive index of 1.60 or more.

**13.** The polymerizable composition for optical components according to claim 12, wherein a difference in refractive index between the epithio compound (1') and the epithio compound (2') is less than 0.10.

**14.** The polymerizable composition for optical components according to claim 1, wherein the polymerizable composition for optical components after being cured has a refractive index of 1.64 to 1.68.

**15.** The polymerizable composition for optical components according to claim 14, wherein the polymerizable composition for optical components after being cured has an Abbe's number of 37 or larger.

**16.** A cured object of the polymerizable composition for optical components according to any one of claims 1 to 15.

**17.** A spectacle lens comprising a lens substrate that includes the cured object according to claim 16.

**INTERNATIONAL SEARCH REPORT**

International application No.
**PCT/JP2022/048205**

**A. CLASSIFICATION OF SUBJECT MATTER**

***G02B 1/04***(2006.01)i; ***C08G 75/08***(2006.01)i; ***G02C 7/00***(2006.01)i
FI: G02B1/04; G02C7/00; C08G75/08

According to International Patent Classification (IPC) or to both national classification and IPC

**B. FIELDS SEARCHED**

Minimum documentation searched (classification system followed by classification symbols)

G02B1/04; C08G75/08; G02C7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2005-281527 A (HOYA CORP.) 13 October 2005 (2005-10-13) paragraphs [0011], [0012], [0020], [0031]-[0036], examples 3, 5 | 1-3, 6-7, 10-17 |
| Y | paragraphs [0011], [0012], [0020], [0031]-[0036], examples 3, 5 | 4-5, 8-15 |
| Y | JP 2000-109478 A (MITSUBISHI GAS CHEM. CO., INC.) 18 April 2000 (2000-04-18) paragraphs [0001]-[0004], [0015]-[0017](18) | 4-5, 8-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/048205**

| Patent document cited in search report | Publication date (day/month/year) | Patent family member(s) | Publication date (day/month/year) |
|---|---|---|---|
| JP 2005-281527 A | 13 October 2005 | (Family: none) | |
| JP 2000-109478 A | 18 April 2000 | US 2001/0041784 A1<br>paragraphs [0001]-[0004], [0022]-[0024](18)<br>EP 978513 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004002820 A